# EUROPÄISCHE PATENTANMELDUNG

(11) **EP 1 897 450 A1**
(43) Veröffentlichungstag der Anmeldung: **12.03.2008**
(21) Anmeldenummer: 07017288.7
(22) Anmeldetag: 04.09.2007
(51) Int. Cl.: A23L 1/30, A61K 31/385, A61K 33/24, A61K 45/06

(54) **Alpha-Liponsäure enthaltende Zusammensetzung**

(30) Priorität: 07.09.2006 DE 202006013847 U
(71) Anmelder: Dr. Gerhard Mann Chem.-pharm. Fabrik GmbH, 13578 Berlin (DE)
(72) Erfinder: Bellmann, Günther, 13593 Berlin (DE); Claus-Herz, Gudrun, 14167 Berlin (DE)
(74) Vertreter: Maiwald Patentanwalts GmbH

(57) **Zusammenfassung**

Die vorliegende Erfindung betrifft eine α-Liponsäure enthaltende Zusammensetzung, geeignet zur Behandlung der diabetischen Retinopathie, wobei die Zusammensetzung Chromverbindungen und Isoflavon(e) umfasst.

## Beschreibung

Die vorliegende Erfindung betrifft eine α-Liponsäure enthaltende Zusammensetzung. Die α-Liponsäure enthaltende Zusammensetzung kann beispielsweise als Nahrungsergänzungsmittel oder als Mittel zur ergänzenden bilanzierten Diät bei Erkrankungen oder Zuständen des Auges, insbesondere bei diabetischer Retinopathie verwendet werden.

Diabetes mellitus, auch Zuckerkrankheit genannt, ist eine Erkrankung mit äußerst komplexen Syndromen und Ursprüngen. Beim Diabetes mellitus treten infolge der erhöhten Blutzuckerkonzentration im Verlauf der Krankheit zunehmend Veränderungen der Blutgefäße auf. Diese Gefäßschäden sind die Ursache für weitreichende Schäden von Gewebe und Organen, wie Nieren, Nerven und des Herz-Kreislauf-Systems, an denen viele Diabetiker leiden. Insbesondere sind auch die Augen von den Folgeschäden des Diabetes mellitus betroffen. Gefäßveränderungen der Netzhaut des Auges können zu Gefäßverschlüssen und damit zu Mangelversorgung der Sehzellen, oder zu Veränderungen der Gefäßwände führen. Eine der Folgeerkrankungen des Auges, die diabetische Netzhauterkrankung oder diabetische Retinopathie, ist die häufigste Ursache für Erblindungen im Erwachsenenalter, so verlieren jährlich etwa 6000 Diabetiker auf diese Weise ihr Augenlicht. Problematisch ist hierbei vor allem, dass die das Sehvermögen zerstörenden Veränderungen den Betroffenen erst dann bewusst werden, wenn die krankhaft neuen Blutgefäße die Sehzellen in der Makula, d.h. der Netzhautmitte, schädigen bzw. es zu Einblutungen in den Glaskörper kommt.

Im Stand der Technik sind Liponsäure enthaltende Zusammensetzungen zur Behandlung der diabetischen Retinopathie bekannt. Im Stand der Technik bekannte Liponsäurepräparate enthalten weiterhin häufig hochdosierte Zusammensetzungen verschiedener antioxidativ wirkender Vitamine, die die Netzhaut vor einer oxidativen Zellschädigung schützen sollen.

Diese Präparate haben jedoch den Nachteil, dass sie für eine Dauertherapie, bzw. eine zeitlich unbegrenzte Prophylaxe nicht geeignet sind. Ein weiterer Nachteil handelsüblicher Präparate besteht darin, dass diese nicht auf eine Versorgung des Auges bei diabetischer Retinopathie abgestimmt sind.

Im Stand der Technik sind weiterhin Kombinationen von Wirkstoffen mit α-Liponsäure in Zusammensetzungen an sich bekannt. So beschreibt beispielsweise die EP 0 994 705 B1 therapeutische oder diätetische Zusammensetzungen umfassend essentielle Fettsäuren und α-Liponsäure zur Behandlung von Diabetes, Insulinresistenz und diabetischen Komplikationen wie Retinopathien. Nachteilig hierbei ist jedoch, dass diese Fettsäuren enthaltenden Zusammensetzungen auf die Behandlung von mit Diabetes zusammenhängenden Entzündungsvorgängen gerichtet sind, jedoch nicht auf die diabetische Retinopathie.

Es ist daher die Aufgabe der vorliegenden Erfindung eine Zusammensetzung zur Verfügung zu stellen, die wenigstens einen der vorgenannten Nachteile des Standes der Technik überwindet. Insbesondere ist es die Aufgabe der vorliegenden Erfindung eine Zusammensetzung zur Verfügung zu stellen, die zur Anwendung bei diabetischer Retinopathie geeignet ist.

Diese Aufgabe wird gelöst durch eine α-Liponsäure enthaltende Zusammensetzung, geeignet zur Behandlung der diabetischen Retinopathie, wobei die Zusammensetzung Chromverbindungen und Isoflavon(e) umfasst.

Im Sinne dieser Verbindung bedeutet der Begriff "α-Liponsäure", auch Thioctsäure genannt, racemische α-Liponsäure oder jede Mischung von deren R- und S-Isomeren, die reduzierte Form der α-Liponsäure, Dihydroliponsäure, sowie pharmazeutisch verträgliche Salze der vorgenannten Verbindungen.

Es hat sich überraschend gezeigt, dass das Auge bei einer diabetischen Retinopathie durch die optimierte Kombination von Chromverbindungen und Isoflavonen mit α-Liponsäure deutlich gestärkt werden kann. Es hat sich weiterhin überraschend gezeigt, dass die erfindungsgemäße Kombination von Chromverbindungen, Isoflavonen und α-Liponsäure einen verbesserten vorbeugenden Schutz der Netzhaut bewirken kann.

Es konnte festgestellt werden, dass der Zusatz von Isoflavonen eine maßgebliche Verbesserung der Wirkung der α-Liponsäure enthaltenden Zusammensetzung bei diabetischer Retinopathie bewirken kann.

Weiterhin scheint insbesondere auch die Verwendung von Chromverbindungen in Verbindung mit α-Liponsäure und Isoflavonen eine überraschend gute synergistische Wirkung bei der Behandlung der diabetischen Retinopathie zu bewirken.

Unter "Stoff" sind im Sinne dieser Erfindung die Stoffe zu verstehen, die die Zusammensetzung aufweist, insbesondere α-Liponsäure, Isoflavone, Chromverbindungen, Vitamin C, Vitamin E, Pyridoxin, Thiamin, Niacinamid sowie mikrokristalline Cellulose, Siliciumdioxid, Stearinsäuren und Stearate, vorzugsweise Magnesiumstearat.

In bevorzugten Ausführungsformen umfasst die α-Liponsäure enthaltende Zusammensetzung wenigstens ein Vitamin, vorzugsweise wenigstens ein Vitamin ausgewählt aus der Gruppe umfassend Vitamin C, Vitamin E, Pyridoxin, Thiamin und/oder Niacinamid.

Pyridoxin, Thiamin und/oder Niacinamid werden ebenfalls als Vitamin B6, Vitamin B1 und Vitamin B3 bezeichnet.

Eine Ergänzung der α-Liponsäure enthaltenden Zusammensetzung mit Vitaminen ausgewählt aus der Gruppe umfassend Vitamin C, Vitamin E, Pyridoxin, Thiamin und/oder Niacinamid kann einen weiteren Vorteil der Zusammensetzung durch eine Verbesserung der antioxidativen Eigenschaften bewirken. Weiterhin kann eine Ergänzung mit Vitaminen ausgewählt aus der Gruppe umfassend Vitamin C, Vitamin E, Pyridoxin, Thiamin und/oder Niacinamid den diabetischen Spätkomplikationen entgegenwirken. Eine Ergänzung der Zusammensetzung mit diesen Vitaminen kann einen umfassenden Schutz des Auges bei einer diabetischen Retinopathie zur Verfügung stellen, die eine Schädigung des Auges und insbesondere der Netzhaut verhindern. Somit kann das Auge vor einer Entstehung einer diabetischen Retinopathie verbessert geschützt werden, wie auch bei einer bereits bestehenden diabetischen Retinopathie die Funktionsfähigkeit der Netzhaut unterstützt werden.

Im Sinne dieser Erfindung ist der Begriff "Isoflavon" gleichbedeutend mit "Isoflavonoid", wie Isoflavone auch bezeichnet werden. Bei den erfindungsgemäßen Isoflavonen oder Isoflavonoiden handelt es sich um Verbindungen, die ein Isoflavon-Grundgerüst aufweisen. Dieses Isoflavon-Grundgerüst kann unterschiedliche funktionelle Gruppen aufweisen. Bevorzugte Isoflavone sind ausgewählt aus der Gruppe umfassend Genistein, Daidzein und/oder Glycitein, besonders bevorzugt ausgewählt aus der Gruppe umfassend Genistein und/oder Daidzein. Bevorzugte erfindungsgemäße Zusammensetzungen können ein Isoflavon, vorzugsweise ausgewählt aus der Gruppe umfassend Genistein, Daidzein und/oder Glycitein enthalten, oder Isoflavone können in Form von Gemischen, wie sie beispielsweise in Pflanzen und/oder Pflanzenextrakten vorliegen, enthalten sein. Die erfindungsgemäße Zusammensetzung kann Isoflavone in Glykosid- und/oder Aglykonform enthalten.

Vorzugsweise enthält die Zusammensetzung Isoflavon(e) in Form von Extrakt(en). Unter dem Begriff "Extrakt" werden im Sinne dieser Erfindung Auszüge von flüssiger oder trockener Beschaffenheit von Pflanzen oder deren Teilen, die beispielsweise unter Verwendung von Wasser, organischen Lösemitteln oder überkritischen Gasen gewonnen werden, verstanden. Bevorzugt sind Isoflavone enthaltende Extrakte aus Leguminosen, vorzugsweise ausgewählt aus der Gruppe umfassend Soja, Linsen, Erbsen und/oder Rotklee. Bevorzugt sind Extrakte von trockener Beschaffenheit, die vorzugsweise aus den frischen oder getrockneten Leguminosen erhältlich sind. Besonders bevorzugt sind Extrakte erhältlich aus Soja, insbesondere erhältlich aus Sojabohnen und/oder Sojasprossen.

Ohne auf eine bestimmte Theorie festgelegt zu sein, wird angenommen, dass die in der erfindungsgemäßen Zusammensetzung enthaltenen Isoflavone einen maßgeblichen Einfluss auf die positiven Eigenschaften der Zusammensetzung bei diabetischer Retinopathie haben. Insbesondere konnte festgestellt werden, dass die in der Zusammensetzung enthaltenen Isoflavone einen positiven Einfluss auf die Neovaskularisation aufweisen können.

Die Kombination der α-Liponsäure enthaltenden Zusammensetzung mit Isoflavonen und Chrom kann eine überraschende Verbesserung der Wirkung der erfindungsgemäßen Zusammensetzung zur Verfügung stellen. So scheint insbesondere die Verwendung von Isoflavonen in Kombination mit Chrom eine überraschende synergistische Wirkung der α-Liponsäure enthaltenden Zusammensetzung bei der Behandlung einer diabetischen Retinopathie zu bewirken.

Ein besonderer Vorteil der erfindungsgemäßen Zusammensetzung kann dadurch verwirklicht werden, dass diese eine Kombination von α-Liponsäure mit Chromverbindungen und Isoflavonen zur Verfügung stellt, die in vorteilhafter Weise eine ausgewogene Versorgung des Auges zur Verfügung stellen kann. Die gezielte Zufuhr dieser Stoffe kann vorteilhafterweise dazu beitragen, das Auge und insbesondere die Retina vor der Entstehung einer diabetischen Retinopathie zu schützen oder deren Fortschreiten zu verlangsamen.

In bevorzugten Ausführungsformen der Zusammensetzung liegt das Gewichtsverhältnis von Genistein zu Daidzein im Bereich von ≥ 1:10 bis ≤ 10:1, vorzugsweise im Bereich von ≥ 1:1 1 bis ≤ 10:1, bevorzugt im Bereich von ≥1,5:1 bis ≤ 5:1, mehr bevorzugt im Bereich von ≥ 2:1 bis ≤ 4:1, besonders bevorzugt im Bereich von ≥ 2,5:1 bis ≤ 3,5:1. Hierbei ist die Angabe "Genistein" so zu verstehen, dass das Gewichtsverhältnis bezogen auf die Aglykone berechnet ist, unabhängig davon, ob in der Zusammensetzung die Isoflavone in Form der Aglykone und/oder Glykoside vorliegen.

Es konnte festgestellt werden, dass insbesondere die erfindungsgemäß bevorzugten Gewichtsverhältnisse der Isoflavone zu einer Verbesserung der Wirkung der erfindungsgemäßen Zusammensetzung beitragen können.

In bevorzugten Ausführungsformen umfasst die α-Liponsäure enthaltende Zusammensetzung bezogen auf eine Tagesdosis der Zusammensetzung Isoflavon(e) im Bereich von ≥ 5 mg bis ≤ 100 mg, bevorzugt im Bereich von ≥ 10 mg bis ≤ 50 mg, mehr bevorzugt im Bereich von ≥ 20 mg bis ≤ 40 mg, besonders bevorzugt im Bereich von ≥ 25 mg bis ≤ 35 mg. Es kann weiterhin bevorzugt sein, dass die α-Liponsäure enthaltende Zusammensetzung, bezogen auf eine Einzeldosis, Genistein im Bereich von ≥ 5 mg bis ≤ 30 mg, bevorzugt im Bereich von ≥ 10 mg bis ≤ 20 mg, mehr bevorzugt im Bereich von ≥ 12 mg bis ≤ 18 mg, besonders bevorzugt im Bereich von ≥ 14 mg bis ≤ 16 mg aufweist.

In weiterhin bevorzugten Ausführungsformen enthält die Zusammensetzung α-Liponsäure im Bereich von ≥ 30 mg bis ≤ 600 mg, bevorzugt im Bereich von ≥ 50 mg bis ≤ 250 mg, mehr bevorzugt im Bereich von ≥ 100 mg bis ≤ 200 mg, besonders bevorzugt im Bereich von ≥ 140 mg bis ≤ 160 mg.

Weiterhin weist die α-Liponsäure enthaltende Zusammensetzung Chromverbindungen auf. Geeignete Chromverbindungen können beispielsweise ausgewählt sein aus der Gruppe umfassend Chrompicolinat und/oder Chromsulfat. Vorzugsweise weist die erfindungsgemäße Zusammensetzung Chromverbindungen in Form von Chromsalzen auf. Chromsalze sind vorzugsweise gut wasserlöslich und/oder gut verträglich. In verwendbaren Chromsalzen kann Chrom in unterschiedlichen Oxidationsstufen vorliegen. Bevorzugt unter den Chromsalzen sind Chrom(III)salze, insbesondere ausgewählt aus der Gruppe umfassend Chrom(III)chlorid und/oder dessen Hydrate, insbesondere bevorzugt ist Chrom(III)chlorid-Hexahydrat.

Es hat sich überraschend gezeigt, dass insbesondere die Verwendung von Chrom(III)chlorid-Hexahydrat die Aufnahme des Chroms verbessern kann. Es wird vermutet, dass die Versorgung der Retina mit Chrom insbesondere durch die Verwendung von Chrom(III)chlorid-Hexahydrat verbessert wird.

In noch bevorzugten Ausführungsformen enthält die Zusammensetzung, bezogen auf eine Tagesdosis der Zusammensetzung, Chromverbindungen, insbesondere Chromsalze, im Bereich von ≥ 0,02 mg bis ≤ 1 mg, bevorzugter im Bereich von ≥ 0,04 mg bis ≤ 0,5 mg, mehr bevorzugt im Bereich von ≥ 0,05 mg bis ≤ 0,2 mg, besonders bevorzugt im Bereich von ≥ 0,1 mg bis ≤ 0,15 mg.

Durch die erfindungsgemäße Zusammensetzung kann ein umfassender Schutz des Auges vor einer diabetischen Schädigung und insbesondere vor einer diabetischen Retinopathie zur Verfügung gestellt werden. Ein weiterer Vorteil der erfindungsgemäßen Zusammensetzung kann durch die enthaltenen Vitamine zur Verfügung gestellt werden, die den umfassenden Schutz des Auges vor einer Entstehung einer diabetischen Retinopathie weiter verbessern können. Die Vitamine können beispielsweise in Form ihrer Säure vorkommen oder in Form von Salzen oder anderen geeigneten Verbindungen, wobei grundsätzlich alle Verbindungen einsetzbar sind, solange sie verträglich sind. Beispielsweise kann die Zusammensetzung Vitamin C in Form von Ascorbinsäure, Vitamin E in Form von Vitamin E-acetat, Pyridoxin in Form von Pyridoxin-Hexachlorid, Thiamin in Form von Thiaminmononitrat und/oder Niacinamid aufweisen.

Weiterhin hat sich herausgestellt, dass durch eine zusätzliche Verwendung von wenigstens einem Vitamin ausgewählt aus der Gruppe umfassend Vitamin C, Vitamin E, Pyridoxin, Thiamin und/oder Niacinamid eine weitere positive Verbesserung der Zusammensetzung erzielt werden kann. In weiteren bevorzugten Ausführungsformen der Zusammensetzung enthält diese, bezogen auf eine Tagesdosis, Vitamin C im Bereich von ≥ 10 mg bis ≤ 400 mg, bevorzugt im Bereich von ≥ 20 mg bis ≤ 300 mg, mehr bevorzugt im Bereich von ≥ 50 mg bis ≤ 250 mg, besonders bevorzugt im Bereich von ≥ 80 mg bis ≤ 120 mg.

Weiterhin können Ausführungsformen bevorzugt sein, bei welchen die Zusammensetzung weiterhin Vitamin E enthält, beispielsweise kann die Zusammensetzung Vitamin E im Bereich von ≥ 1 mg bis ≤ 50 mg, bevorzugt im Bereich von ≥ 2 mg bis ≤ 40 mg, mehr bevorzugt im Bereich von ≥ 5 mg bis ≤ 25 mg, besonders bevorzugt im Bereich von ≥ 8 mg bis ≤ 12 mg enthalten.

Vorzugsweise enthält die Zusammensetzung weiterhin, bezogen auf eine Tagesdosis der Zusammensetzung, Pyridoxin in Bereich von ≥ 0,5 mg bis ≤ 10 mg, bevorzugt im Bereich von ≥ 1 mg bis ≤ 5 mg, mehr bevorzugt im Bereich von ≥ 1,5 mg bis ≤ 4 mg, besonders bevorzugt im Bereich von ≥ 1,8 mg bis ≤ 2,2 mg, und/oder Thiamin im Bereich von ≥ 0,2 mg bis ≤ 10 mg, bevorzugt im Bereich von ≥ 0,5 mg bis ≤ 4 mg, mehr bevorzugt im Bereich von ≥ 1 mg bis ≤ 3 mg, besonders bevorzugt im Bereich von ≥ 1,4 mg bis ≤ 1,6 mg, und/oder Niacinamid im Bereich von ≥ 1 mg bis ≤ 50 mg, bevorzugt im Bereich von ≥ 5 mg bis ≤ 40 mg, mehr bevorzugt im Bereich von ≥ 10 mg bis ≤ 30 mg, besonders bevorzugt im Bereich von ≥ 15 mg bis ≤ 25 mg.

In besonders bevorzugten Ausführungsformen umfasst die Zusammensetzung, bezogen auf eine Tagesdosis der Zusammensetzung:
a. ≥ 30 mg bis ≤ 600 mg, bevorzugt ≥ 50 mg bis ≤ 250 mg, mehr bevorzugt ≥ 100 mg bis ≤ 200 mg, besonders bevorzugt ≥ 140 mg bis ≤ 160 mg α-Liponsäure; und/oder
b. ≥ 5 mg bis ≤ 100 mg, bevorzugt ≥ 10 mg bis ≤ 50 mg, mehr bevorzugt ≥ 20 mg bis ≤ 40 mg, besonders bevorzugt ≥ 25 mg bis ≤ 35 mg Isoflavon(e); und/oder
c. ≥ 0,02 mg bis ≤ 1 mg, bevorzugt ≥ 0,04 mg bis ≤ 0,5 mg, mehr bevorzugt ≥ 0,05 mg bis ≤ 0,2 mg, besonders bevorzugt ≥ 0,1 mg bis ≤ 0,15 mg Chromverbindungen; und/oder
d. ≥ 10 mg bis ≤ 400 mg, bevorzugt ≥ 20 mg bis ≤ 300 mg, mehr bevorzugt ≥ 50 mg bis ≤ 250 mg, besonders bevorzugt ≥ 80 mg bis ≤ 120 mg Vitamin C; und/oder
e. ≥ 1 mg bis ≤ 50 mg, bevorzugt ≥ 2 mg bis ≤ 40 mg, mehr bevorzugt ≥ 5 mg bis ≤ 25 mg, besonders bevorzugt ≥ 8 mg bis ≤ 12 mg Vitamin E; und/oder
f. ≥ 0,5 mg bis ≤ 10 mg, bevorzugt ≥ 1 mg bis ≤ 5 mg, mehr bevorzugt ≥ 1,5 mg bis ≤ 4 mg, besonders bevorzugt ≥ 1,8 mg bis ≤ 2,2 mg Pyridoxin; und/oder
g. ≥ 0,2 mg bis ≤ 10 mg, bevorzugt ≥ 0,5 mg bis ≤ 4 mg, mehr bevorzugt ≥ 1 mg bis ≤ 3 mg, besonders bevorzugt ≥ 1,4 mg bis ≤ 1,6 mg Thiamin; und/oder
h. ≥ 1 mg bis ≤ 50 mg, bevorzugt ≥ 5 mg bis ≤ 40 mg, mehr bevorzugt ≥ 10 mg bis ≤ 30 mg, besonders bevorzugt ≥ 15 mg bis ≤ 25 mg Niacinamid.

In einer ganz besonders bevorzugten Ausführungsform der Zusammensetzung umfasst die Zusammensetzung, bezogen auf eine Tagesdosis 150 mg α-Liponsäure, 30 mg Isoflavon(e), 0,12 mg Chromverbindung, 100 mg Vitamin C, 10 mg Vitamin E, 2 mg Pyridoxin, 1,5 mg Thiamin und/oder 20 mg Niacinamid.

In einer weiterhin besonders bevorzugten Ausführungsform der Zusammensetzung umfasst die Zusammensetzung, bezogen auf eine Einzeldosis 75 mg α-Liponsäure, 15 mg Isoflavon(e), 0,06 mg Chromverbindung, 50 mg Vitamin C, 5 mg Vitamin E, 1 mg Pyridoxin, 0,75 mg Thiamin und/oder 10 mg Niacinamid.

Die α-Liponsäure enthaltende Zusammensetzung weist Chrom in Form von dessen Salzen oder anderer geeigneter Verbindungen auf, vorzugsweise in Form von Chrom(III)chlorid. Angaben der Gehalte an Chrom sind bezogen auf den Gehalt an Chrom und nicht auf den Gehalt an Chrom(III)chlorid oder anderen Chromverbindungen.

Unter einer Tagesdosis wird im Sinne dieser Anmeldung die Menge der Zusammensetzung verstanden, die pro Tag verabreicht wird. Unter einer Einzeldosis wird im Sinne dieser Erfindung eine Verabreichungseinheit der α-Liponsäure enthaltenden Zusammensetzung verstanden. Eine Einzeldosis entspricht beispielsweise einer Tablette, einer Filmtablette, einem Dragee, einer Kapsel oder einer einzelnen Verabreichungseinheit einer anderen Darreichungsform, wie Pulver oder Granulat. Eine Tagesdosis und/oder Einzeldosis ist hierbei vorzugsweise auf eine oder mehrere gleiche oder unterschiedliche Darreichungsformen verteilt, wobei die Tagesdosis vorzugsweise mehreren Einzeldosen entspricht. Vorzugsweise entspricht eine Tagesdosis beispielsweise zwei Einzeldosen.

Die α-Liponsäure enthaltende Zusammensetzung kann fest-, flüssig und gelförmig vorliegen, vorzugsweise liegt die Zusammensetzung in Darreichungsformen ausgewählt aus der Gruppe umfassend Tabletten, Filmtabletten, Dragees, Pulver, Granulat, Lösungen und/oder Brausetabletten vor. Bevorzugt liegt die α-Liponsäure enthaltende Zusammensetzung in Form von Tabletten, insbesondere in Form einer Filmtablette vor. Vorzugsweise umfasst die Tablette einen Tablettenkern, der mit einem Filmüberzug versehen sein kann.

In bevorzugten Ausführungsformen liegt die α-Liponsäure enthaltende Zusammensetzung in Form einer festförmigen Darreichungsform vor, wobei der Kern der Darreichungsform bevorzugt ≥ 200 mg bis ≤ 800 mg, vorzugsweise ≥ 400 mg bis ≤ 600 mg, bevorzugt ≥ 500 mg bis ≤ 540 mg und besonders bevorzugt ≥ 510 mg bis ≤ 530 mg ausmacht. Die Filmmenge liegt hierbei vorzugsweise in einem Bereich von ≥ 5 mg bis ≤ 100 mg, vorzugsweise in einem Bereich von ≥ 20 mg bis ≤ 60 mg, besonders bevorzugt in einem Bereich von ≥ 30 mg bis ≤ 50 mg pro Tablettenkern. In einer anderen Ausführungsform macht der Kern der Darreichungsform bevorzugt ≥ 200 mg bis ≤ 800 mg, vorzugsweise ≥ 520 mg bis ≤ 680 mg, bevorzugt ≥ 580/mg bis ≤ 620 mg und besonders bevorzugt ≥ 590 mg bis 610 aus.

Die α-Liponsäure enthaltende Zusammensetzung, insbesondere der Tablettenkern, weist gegebenenfalls weitere Hilfsstoffe aus. Hilfsstoffe sind vorzugsweise ausgewählt aus der Gruppe umfassend Cellulose, bevorzugt mikrokristalline Cellulose, Lactose, Calciumphosphat, Siliciumdioxid, Stearinsäuren und/oder Stearat, vorzugsweise Magnesiumstearat. Weiterhin kann die α-Liponsäure enthaltende Zusammensetzung Überzugsmittel, auch bezeichnet als Filmbildner oder Coating-Stoffe, aufweisen. Beispielsweise kann der Filmüberzug Stoffe ausgewählt aus der Gruppe umfassend Hydroxypropylmethylcellulose, Talkum, Stearinsäure, Triglyceride, insbesondere mittelkettige Triglyceride, Titandioxid und/oder Farbstoffe, bevorzugt ausgewählt aus der Gruppe umfassend Eisenoxid gelb, Eisenoxid schwarz, Brilliantblau, und/oder Indigokarmin, aufweisen. In bevorzugten Ausführungsformen sind die Farbstoffe in Form von Aluminiumlack verwendbar.

Die Herstellung der Darreichungsformen, beispielsweise Tablette, Filmtablette, Dragee oder Kapsel erfolgt vorzugsweise nach üblichen Verfahren, beispielsweise durch Kompaktierung, Granulierung und/oder Abfüllen in Gelatinekapseln. Üblicherweise werden zur Herstellung von Tabletten die Tablettenkernbestandteile trocken vermischt und zu Tabletten verpresst. Anschließend wird beispielsweise durch Sprühen oder Lackieren der Filmüberzug aus einer geeigneten Lösung oder Suspension aufgetragen.

In bevorzugten Ausführungsformen beträgt das Gewicht des Tablettenkerns 520 mg, das der beschichteten Tablette 560 mg, der Durchmesser des Tablettenkerns 11,5 mm, die Dicke des Tablettenkerns 5,0 bis 5,2 mm, die Bruchfestigkeit liegt im Bereich von 120 N bis 170 N und/oder der Abrieb ist kleiner als 0,5 Gew.-%, bezogen auf das Gesamtgewicht des Tablettenkerns. In einer anderen bevorzugten Ausführungsform beträgt das Gewicht des Tablettenkerns 600 mg und das der beschichteten Tablette 645 mg.

Bevorzugt ist, dass die Zusammensetzung in Form einer Tablette vorliegt, insbesondere einer Tablette, die im gastro-intestinalen Trakt zerfällt. In bevorzugten Ausführungsformen liegt die Zerfallsgeschwindigkeit des Tablettenkerns in Wasser im Bereich von 10 min bis 20 min, die Zerfallsgeschwindigkeit beschichteten Tablette in Wasser im Bereich von weniger als 30 min.

In bevorzugten Ausführungsformen weist die α-Liponsäure enthaltende Zusammensetzung Isoflavon(e) in Form von Extrakten erhältlich aus Soja, Chrom in Form von Chrom(III)chlorid-Hexahydrat, Vitamin E in Form von Vitamin E-acetat, α-Liponsäure, Vitamin C in Form von Ascorbinsäure, Pyridoxin in Form von Pyridoxinhydrochlorid, Thiamin in Form von Thiaminmononitrat, Niacinamid, mikrokristalline Cellulose, Siliciumdioxid, Stearinsäure und/oder Stearate, beispielsweise Magnesiumstearat auf.

In einer bevorzugten Zusammensetzung umfasst die α-Liponsäure enthaltende Zusammensetzung bezogen auf eine Tagesdosis der Zusammensetzung:
a. 150 mg α-Liponsäure;
b. 30 mg Isoflavon(e)
c. 0,12 mg Chrom;
d. 100 mg Vitamin C;
e. 10 mg Vitamin E;
f. 2 mg Pyridoxin;
g. 1,5 mg Thiamin;
h. 20 mg Niacinamid;
wobei Chrom in Form von Chrom(III)chlorid vorliegt und wobei die Angabe in mg bezogen ist auf das Metall.

In einer noch bevorzugten Ausführungsform umfasst die α-Liponsäure enthaltende Zusammensetzung, bezogen auf eine Einzeldosis der Zusammensetzung:
a. 75 mg α-Liponsäure;
b. 15 mg Isoflavon(e);
c. 0,06 mg Chrom;
d. 50 mg, Vitamin C;
e. 5 mg, Vitamin E;
f. 1 mg, Pyridoxin;
g. 0,75 mg, Thiamin;
h. 10 mg Niacinamid,
wobei Chrom in Form von Chrom(III)chlorid vorliegt und wobei die Angabe in mg bezogen ist auf das Metall.

Die α-Liponsäure enthaltende Zusammensetzung kann zur Herstellung eines Mittels zur diätetischen Verhütung und/oder Behandlung von Zuständen oder Erkrankungen des Auges, bevorzugt bei diabetischer Retinopathie verwendet werden. Die α-Liponsäure enthaltende Zusammensetzung ist insbesondere geeignet als Nahrungsergänzungsmittel oder als Mittel zur ergänzenden bilanzierten Diät. Der Begriff "diätetische Behandlung" ist in der EU-Richtlinie 1999/21/EG näher erläutert.

Insbesondere ist die α-Liponsäure enthaltende Zusammensetzung als Nahrungsergänzungsmittel oder als Mittel zur ergänzenden bilanzierten Diät, insbesondere als Lebensmittel, verwendbar. Bevorzugt ist die α-Liponsäure enthaltende Zusammensetzung zur ergänzenden bilanzierten Diät zur Verhütung von diabetischer Retinopathie verwendbar. Es hat sich gezeigt, dass insbesondere ein positiver Effekt auf die Funktionsfähigkeit des Auges bei Diabetes durch eine Verabreichung im Rahmen einer ergänzenden bilanzierten Diät erzielt werden kann.

### Beispiele für α-Liponsäure enthaltende Zusammensetzungen werden nachstehend angegeben:

Es versteht sich, dass die Darreichungsformen, vorzugsweise ausgewählt aus der Gruppe umfassend Tabletten, Filmtabletten, Dragees und/oder Kapseln übliche zur Formulierung verwendbare Hilfsstoffe aufweisen, so dass in den Beispielen lediglich die enthaltenen Wirkstoffe aufgeführt sein können. Zusätzlich zu den angegebenen Mengen können weiterhin übliche Hilfsstoffe und/oder Stabilitätszuschläge und/oder Produktionszuschläge enthalten sein.

### Beispiel 1

α-Liponsäure enthaltende Zusammensetzung, Tagesdosis umfassend:
- 150 mg α-Liponsäure;
- 30 mg Isoflavone;
- 0,12 mg Chrom;
- 100 mg Vitamin C;
- 10 mg Vitamin E;
- 2 mg Pyridoxin;
- 1,5 mg Thiamin;
- 20 mg Niacinamid,
   wobei die Angabe in mg bezogen ist auf das Metall und wobei Chrom als Chrom(III)chlorid-Hexahydrat vorliegt.

### Beispiel 2

Filmtablette à 560 mg, umfassend:
- 75 mg α-Liponsäure;
- 15 mg Isoflavone;
- 0,06 mg Chrom;
- 50 mg Vitamin C;
- 5 mg Vitamin E;
- 1 mg Pyridoxin;
- 0,75 mg Thiamin;
- 10 mg Niacinamid,
wobei als Hilfsstoffe mikrokristalline Cellulose, Siliciumdioxid und Magnesiumstearat enthalten sind und wobei der Tablettenkern 520 mg ausmacht.

### Beispiel 3

Filmtablette à 645 mg, umfassend:
- 75 mg α-Liponsäure;
- 15 mg Isoflavone;
- 0,06 mg Chrom;
- 50 mg Vitamin C;
- 5 mg Vitamin E;
- 1 mg Pyridoxin;
- 0,75 mg Thiamin;
- 10 mg Niacinamid,
- wobei als Hilfsstoffe mikrokristalline Cellulose, Siliciumdioxid und Magnesiumstearat enthalten sind und wobei der Tablettenkern 600 mg ausmacht.

## Patentansprüche

1. α-Liponsäure enthaltende Zusammensetzung, geeignet zur Behandlung der diabetischen Retinopathie, **dadurch gekennzeichnet, dass** die Zusammensetzung Chromverbindungen und Isoflavon(e) umfasst.

2. Zusammensetzung nach Anspruch 1, **dadurch gekennzeichnet, dass** die Zusammensetzung wenigstens ein Vitamin, vorzugsweise ausgewählt aus der Gruppe umfassend Vitamin C, Vitamin E, Pyridoxin, Thiamin und/oder Niacinamid, aufweist.

3. Zusammensetzung nach Anspruch 1 oder 2, **dadurch gekennzeichnet, dass** die Isoflavone ausgewählt sind aus der Gruppe umfassend Genistein, Daidzein und/oder Glycitein.

4. Zusammensetzung nach einem der vorherigen Ansprüche, **dadurch gekennzeichnet, dass** die Zusammensetzung Isoflavon(e) in Form von Extrakt(en) erhältlich aus Soja umfasst.

5. Zusammensetzung nach einem der vorherigen Ansprüche, **dadurch gekennzeichnet, dass** das Gewichtsverhältnis von Genistein zu Daidzein im Bereich von ≥ 1:10 bis ≤ 10:1, bevorzugt im Bereich von ≥ 1,5:1 bis ≤ 5:1, mehr bevorzugt im Bereich von ≥ 2:1 bis ≤ 4:1, besonders bevorzugt im Bereich von ≥ 2,5:1 bis ≤ 3,5:1 liegt.

6. Zusammensetzung nach einem der vorherigen Ansprüche, **dadurch gekennzeichnet, dass** die Zusammensetzung, bezogen auf eine Tagesdosis der Zusammensetzung, umfasst:
a. ≥ 30 mg bis ≤ 600 mg, bevorzugt ≥ 50 mg bis ≤ 250 mg, mehr bevorzugt ≥ 100 mg bis ≤ 200 mg, besonders bevorzugt ≥ 140 mg bis ≤ 160 mg α-Liponsäure; und/oder
b. ≥ 5 mg bis ≤ 100 mg, bevorzugt ≤ 10 mg bis ≤ 50 mg, mehr bevorzugt ≥ 20 mg bis ≤ 40 mg, besonders bevorzugt ≥ 25 mg bis ≤ 35 mg Isoflavon(e); und/oder
c. ≥ 0,02 mg bis ≤ 1 mg, bevorzugt ≥ 0,04 mg bis ≤ 0,5 mg, mehr bevorzugt ≥ 0,05 mg bis ≤ 0,2 mg, besonders bevorzugt ≥ 0,1 mg bis ≤ 0,15 mg Chromverbindungen; und/oder
d. ≥ 10 mg bis ≤ 400 mg, bevorzugt ≥ 20 mg bis ≤ 300 mg, mehr bevorzugt ≥ 50 mg bis ≤ 250 mg, besonders bevorzugt ≥ 80 mg bis ≤ 120 mg Vitamin C; und/oder
e. ≥ 1 mg bis ≤ 50 mg, bevorzugt ≥ 2 mg bis ≤ 40 mg, mehr bevorzugt ≥ 5 mg bis ≤ 25 mg, besonders bevorzugt ≥ 8 mg bis ≤ 12 mg Vitamin E; und/oder
f. ≥ 0,5 mg bis ≤ 10 mg, bevorzugt ≥ 1 mg bis ≤ 5 mg, mehr bevorzugt ≥ 1,5 mg bis ≤ 4 mg, besonders bevorzugt ≥ 1,8 mg bis ≤ 2,2 mg Pyridoxin; und/oder
g. ≥ 0,2 mg bis ≤ 10 mg, bevorzugt ≥ 0,5 mg bis ≤ 4 mg, mehr bevorzugt ≥ 1 mg bis ≤ 3 mg, besonders bevorzugt ≥ 1,4 mg bis ≤ 1,6 mg Thiamin; und/oder
h. ≥ 1 mg bis ≤ 50 mg, bevorzugt ≥ 5 mg bis ≤ 40 mg, mehr bevorzugt ≥ 10 mg bis ≤ 30 mg, besonders bevorzugt ≥ 15 mg bis ≤ 25 mg Niacinamid.

7. Zusammensetzung nach einem der vorherigen Ansprüche, **dadurch gekennzeichnet, dass** die Zusammensetzung fest-, flüssig und/oder gelförmig vorliegt, vorzugsweise liegt die Zusammensetzung in Darreichungsformen, ausgewählt aus der Gruppe umfassend Tabletten, Filmtabletten, Dragees, Kapseln, Pulver, Granulat, Lösungen und/oder Brausetabletten, vor.

8. Zusammensetzung nach einem der vorherigen Ansprüche, **dadurch gekennzeichnet, dass** die Zusammensetzung in Form einer im gastrointestinalen Trakt zerfallenden Tablette, vorzugsweise Filmtablette, vorliegt.

9. Zusammensetzung nach einem der vorherigen Ansprüche, **dadurch gekennzeichnet, dass** die Zusammensetzung in einer sogenannten festen Darreichungsform vorliegt, wobei der Kern der Darreichungsform ≥ 200 mg bis ≤ 800 mg, vorzugsweise ≥ 400 mg bis ≤ 600 mg, bevorzugt ≥ 500 mg bis ≤ 540 mg und besonders bevorzugt ≥ 510 mg bis ≤ 530 mg ausmacht, oder wobei der Kern der Darreichungsfrom ≥ 200 mg bis ≤ 800 mg, vorzugsweise ≥ 520 mg bis 680 mg, bevorzugt ≥ 580 mg bis 620 mg und besonders bevorzugt ≥ 590 mg bis 610 mg ausmacht.

10. Zusammensetzung nach einem der vorherigen Ansprüche, **dadurch gekennzeichnet, dass** die Zusammensetzung Hilfsstoffe ausgewählt aus der Gruppe umfassend Cellulose, vorzugsweise mikrokristalline Cellulose, Lactose, Calciumphosphat, Siliciumdioxid Stearinsäure und/oder Stearat, vorzugsweise Magnesiumstearat aufweist.

11. Zusammensetzung nach einem der vorherigen Ansprüche, **dadurch gekennzeichnet, dass** die Zusammensetzung Extrakt(e) erhältlich aus der Pflanze und/oder aus Pflanzenteilen von Soja, Chrom(III)chlorid-Hexahydrat, Ascorbinsäure, Vitamin E-acetat, Niacinamid, Pyridoxin-hydrochlorid, Thiamninmononitrat, Niacinamid, mikrokristalline Cellulose, Siliciumdioxid, Stearinsäure und/oder Stearat, vorzugsweise Magnesiumstearat aufweist.

12. Zusammensetzung nach einem der vorherigen Ansprüche, **dadurch gekennzeichnet, dass** die Zusammensetzung, bezogen auf eine Tagesdosis der Zusammensetzung, umfasst:
a. 150 mg α-Liponsäure;
b. 30 mg Isoflavon(e);
c. 0,12 mg Chrom;
d. 100 mg Vitamin C;
e. 10 mg Vitamin E;
g. 2 mg Pyridoxin;
g. 1,5 mg Thiamin;
h. 20 mg Niacinamid,
wobei Chrom in Form von Chrom(III)chlorid vorliegt und wobei die Angabe in mg bezogen ist auf das Metall.

13. Zusammensetzung nach einem der vorherigen Ansprüche, **dadurch gekennzeichnet, dass** die Zusammensetzung, bezogen auf eine Tagesdosis der Zusammensetzung, umfasst:
a. 75 mg α-Liponsäure;
b. 15 mg Isoflavon(e);
c. 0,06 mg Chrom;
d. 50 mg, Vitamin C;
e. 5 mg, Vitamin E;
f. 1 mg, Pyridoxin;
g. 0,75 mg, Thiamin;
h. 10 mg Niacinamid,
wobei Chrom in Form von Chrom(III)chlorid vorliegt und wobei die Angabe in mg bezogen ist auf das Metall.

14. Verwendung einer Zusammensetzung nach einem der vorherigen Ansprüche zur Herstellung eines Mittels zur diätetischen Verhütung und/oder Behandlung von Erkrankungen des Auges, bevorzugt zur Verhütung von diabetischer Retinopathie.

15. Verwendung einer Zusammensetzung nach einem der vorherigen Ansprüche als Nahrungsergänzungsmittel und/oder Mittel zur ergänzenden bilanzierten Diät.

16. Mittel, Nahrungsergänzungsmittel oder Mittel zur ergänzenden bilanzierten Diät, insbesondere Lebensmittel, umfassend Stoffe gemäß einem der vorherigen Ansprüche.
